# EUROPEAN PATENT SPECIFICATION

(11) **EP 1 208 853 B1**
(45) Date of publication and mention of the grant of the patent: **27.05.2009**
(21) Application number: 01128024.5
(22) Date of filing: 26.11.2001
(51) Int. Cl.: A61L 9/20

(54) **Modular ultraviolet sterilization apparatus**
Modulare UV-Sterilisationsvorrichtung
Appareil modulaire de stérilisation par rayons ultraviolets

(30) Priority: 28.11.2000 WO PCT/EP00/11880
(43) Date of publication of application: 29.05.2002
(73) Proprietor: Medical Service S.r.l., 84131 Salerno (IT)
(72) Inventor: Bollini, Susanna, I-20146 Milano (IT)
(74) Representative: Barberi, Vittorio

(56) References cited:
- EP-A- 0 461 310
- DE-A- 19 652 688
- GB-A- 2 215 234
- US-A- 4 210 429
- US-A- 5 225 167
- US-A- 5 894 130
- US-A- 5 997 619

## Description

The present invention relates to an apparatus for sterilising the air of rooms and the like by means of ultraviolet radiation, and more particularly to an improved ultraviolet sterilisation apparatus.

The sterilising and bactericide action of ultraviolet radiation known as UV-C (short waves) having a wavelength between 100 and 280 nanometers is used not only for the disinfection of public and private premises, particularly hospitals and nursing homes, but even in rooms opened to the public and also in homes for hygienic and prevention purposes.

There are known devices providing for the use of low pressure mercury vapour lamps as sources of the ultraviolet radiation, which are directed upward but laterally shielded since the direct exposition to this particular radiation is harmful to the persons.

The need to control the bacterial contamination of the air has largely increased in the last years, particularly whereas this parameter can be critical, to comply to safety norms and also for the increased interest for this factor in risk areas where it can relate to the health of the people or the product shelf life.

Beside the evolution and the diffusion of fixed systems of air control in closed environments (laminar flows, negative/positive pressure rooms, high number of air exchanges per hour, absolute filters, etc.) is increasing the demand for stand alone apparatuses that can replace the centralised systems in those cases where these latter cannot be employed for their high costs of installation and management, for the arisen necessity to rapidly sterilise an environment without requiring building works and structural changes or simply because the need is restricted to some areas and it would not be economically convenient to intervene on the whole system.

Moreover movable or easily replaceable apparatuses are better fit to temporary situations such as, for instance, rooms to be restructured or for a temporarily limited need such as in presence of an infectious patient in Dialysis or Intensive care or even for the domiciliary care of immunocompromised patients, in a medical ward, in dentist's surgery, etc.

In this context there are known stand alone apparatuses that use different means to control some parameters of the environmental air: electrostatic filters for reducing the number of suspended particles (smoke and dusts), activated charcoal filters for reducing gases and unpleasant odours, absolute filters (HEPA or ULPA) or germicides ultraviolet radiation (UV-C) to reduce the bacterial charge of the air.

To avoid the side effects of the direct UV-C radiation on human beings it is often convenient to treat the air in a radiation chamber located inside the apparatus where germicidal tubes are housed and the radiation can be kept under control, forcing the room air to pass through the apparatus in the radiation chamber by means of fans and the like.

However the germicidal tubes require frequent servicing because they are negatively effected by the dust that accumulates on their surface and also because their efficiency decays with the time. The use beyond their useful life must be avoided to guarantee an efficient sterilising action.

Dust also strongly reduces the reflecting properties of the inner walls of the radiation chamber in which the tubes are located with a consequent reduction of the apparatus efficiency.

Proper precautions must also be taken when handling and replacing the tubes since the presence of sebum, dirt and fingerprints on the tube surfaces is another frequent cause of efficiency reduction.

From US5894130 and further details on the sterilising effects of UV radiation, it is known an ultraviolet sterilisation unit having a housing attached to an air heating and cooling system and including two openings into which lamp cartridges are inserted. The lamp cartridge carries ultraviolet lamps operating at a frequency capable of sterilising air within the system. The cartridges are configured to automatically de-energise the lamps when a lamp cartridge is removed from the housing. When the sterilisation unit is a multiple lamp system, upon removal of one of the lamp cartridge is removed all lamps are de-energised, with the de-energising of the lamps occurring before a user will view the lamp. The disclosed system is applicable to sterilisation units which are inserted within the air ducts of forced air heating and cooling systems.

It is therefore desirable a system that allows to avoid the above discussed risks and disadvantages, and is capable to eliminate the problems associated with the dust and the excessive ageing of the tubes, that has means to increase the efficacy of the radiation like UV-C reflectors, ensuring a proper, standardised servicing of the apparatus. According to the invention, this is made possible by the use of packages assembled in advance, that cannot be modified and where the inner components cannot be even touched by the operator and most of all is completely safe, simple and fast to be carried out.

An object of the invention is to realise an apparatus for sterilising the ambient air in a closed environment that allows a safe use of the UV-C radiation in inhabited areas, allowing to fully exploit the germicidal properties of the device without undesired collateral effect, and restoring the previous characteristics of the apparatus at every servicing by replacing the germicidal tubes and the reflective walls of the radiation chamber, which operation can be precisely scheduled (since according to an advantageous embodiment of the invention, it is the device itself that indicates the right time interval and time expiration) and kept totally safe and carried out directly by the user without requiring skilled technician personnel with consequent advantages in terms of practicality, economy and reduction of the problems of post-sale technical assistance.

The above objects are achieved by means of the invention which consists of an ultraviolet sterilisation unit as claimed in claim 1.

Additional advantageous features are the subject of the dependent claims.

The apparatus of the invention comprises two main components that are detachable from one another: a supporting frame or housing and a replaceable (and disposable) component or cartridge. The housing or supporting frame incorporates means for fixing the apparatus to a wall or to a pedestal, electric connections, a general control circuitry preferably realised with one or more electronic cards, with control and adjustment buttons, switches and indicator lights, at least a fan and a support for a dust filter for removing the coarser particles from the air flow.

The supporting frame further incorporates an arrangement for securing the cartridge, this latter comprising an envelope or housing of plastics, cardboard, wood or any low cost material that is stiff enough, internally covered or lined by a film of reflecting aluminium to increase the efficiency of the UV radiation.

The housing contains at least a germicidal tube and has two openings for the air entry and exit, such openings being provided with means that allow the passage of the air but stops the germicidal radiation, such as for instance a dark filter or an activated charcoal filter, an optic labyrinth, etc. and a plurality of electric contacts for one or more UV lamp disposed inside the housing and also a system (comprising for example a microchip, a magnetised strip and so on) capable of recording and counting the time of use of the apparatus, as well to supply data for identifying the cartridge.

The invention will now be described with reference to preferred embodiments thereof, together with the attached drawings, in which:
Fig. 1 is a perspective view of a sterilising apparatus according to the invention;
Fig. 2 is longitudinal cross section of the apparatus shown in Fig. 1;
Fig. 3 is a top view of the replaceable lamp cartridge of the apparatus shown in Figures 1 and 2;
Fig. 4 is a cross section view along the line IV-IV of Fig. 3, illustrating further details of the replacement cartridge;
Figs. 5 and 6 are cross section views illustrating another embodiment of a sterilising apparatus according to the invention.

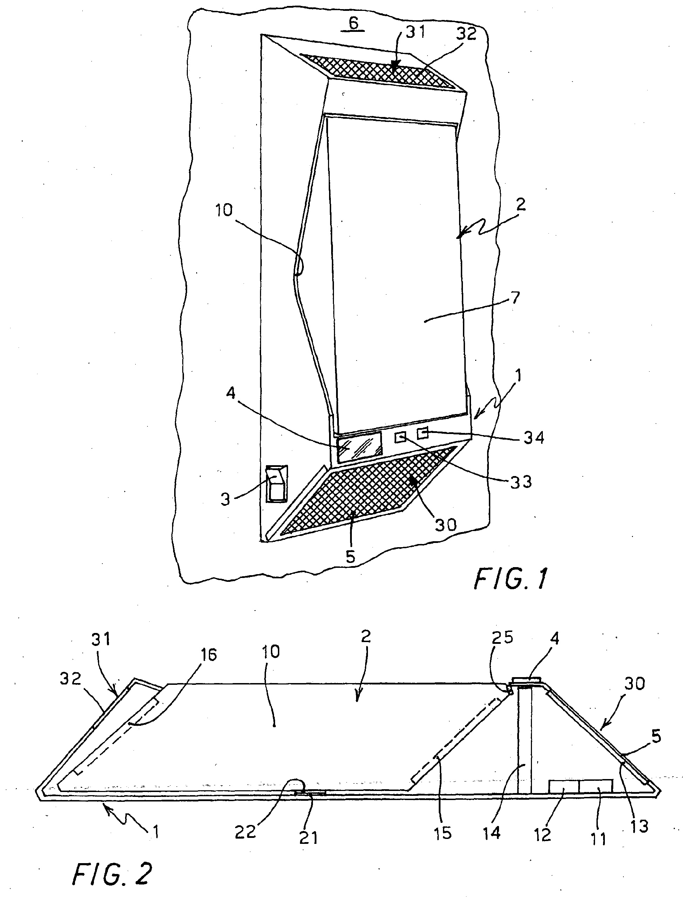

With reference to Figures 1 and 2, a sterilisation apparatus according to the invention comprises a support frame or housing 1 provided with an aperture 10, and a disposable lamp cartridge 2 at least a portion of which can be inserted into and/or fastened to the housing. For sake of clarity, Fig. 2 does not show details of the cartridge. The support frame 1 which has preferably an elongated shape, is further provided with an inlet opening 30 protected by a grid 5 at one end and an outlet opening 31 at the opposed end, this latter opening being protected by a grid 32 and preferably having a surface smaller than the cross section of the housing so as to increase the speed of the outgoing sterilised air thus preventing air stifling near the outlet opening. As schematically shown in Fig. 2, a chamber or space 35 is provided between the cartridge 2 and the wall of the support frame 1 containing the opening 31 and the grid 32.

The support frame 1 is formed as supporting frame incorporating means (not shown) for fixing the apparatus to a wall 6, and houses a power supply 11 coupled to the mains or other electric source for feeding the whole unit, and a general control circuitry 12 preferably realised with one or more electronic cards. The housing 1 further contains a first coarse filter 13 covering the inlet opening 30 of the housing together with the protection grid 5 through which the air enters the apparatus sucked by one or more (axial) fans 14 located inside the housing and interposed between such filter 13 and the aperture 10. The coarse filter 13 is mainly provided for preventing dust particles from entering the apparatus.

The support frame or housing 1 further incorporates control and adjustment buttons, such as a ON/OFF switch 3, a display 4 and indicator lights such as 33 and 34, and an arrangement for securing the cartridge 2 to the housing as will be illustrated in detail later. In the illustrated embodiment the switch 3 is located on a side of the housing and the display 4 is positioned on a narrow wall of the housing adjacent the cartridge and carrying electrical connections 25 as well as blocking means on the surface facing the inside space of the housing. Advantageously the display shows the operating hours of a cartridge, i.e. the time for which the lamps in the presently inserted cartridge has been operated, so that the operator can immediately realise when a cartridge has to be replaced.

Advantageously the flat panel 7 forming the visible surface of the cartridge 2 when this latter is fitted within the housing 1, is adapted to carry an advertising message and the like, e.g. by applying an adhesive sheet over its surface.

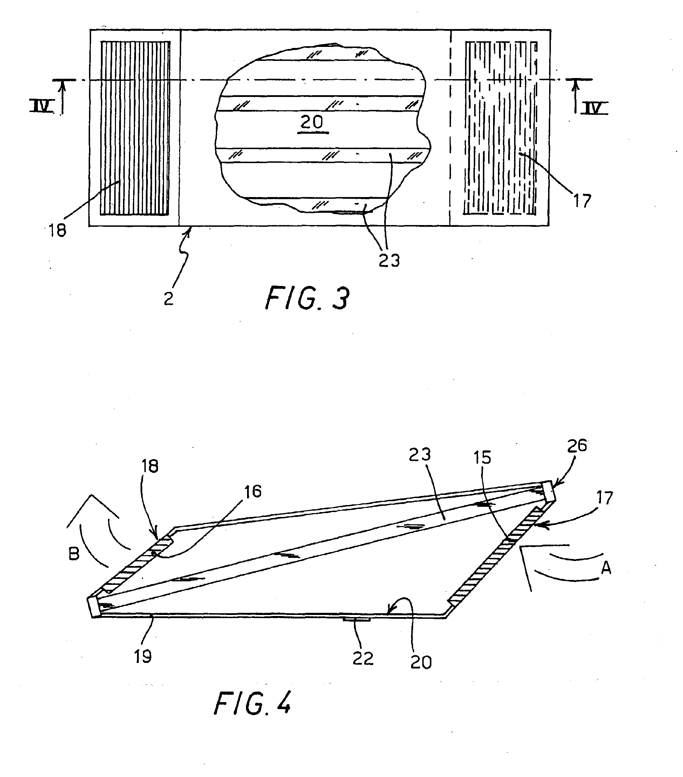

With reference to Figures 3 and 4, the lamp cartridge 2 comprises an envelope 19 made of a low-cost relatively-stiff material such as plastics, cardboard, wood or the like, having a reflective inner surface 20 to increase the sterilising efficiency of the UV radiation. According to a presently preferred embodiment, such envelope is internally lined or coated by a film of reflecting material such as aluminium.

The cartridge 2 further contains at least one germicidal tube 23 and has two openings 17, 18 for the entry and exit of an air flow, schematically shown by arrows A and B, respectively. At least one of such openings is provided with means that allow the passage of the air but stops the germicidal radiation, such as for instance a dark filter or an activated charcoal filter and similar devices. In the illustrated embodiment, the replaceable cartridge 2 provides for two UV stopping devices or filters 15, 16 one at each end of the cartridge, which filters are discarded together with the cartridge when this latter is replaced.

Advantageously, as shown in the figures, the lamp or each lamp is a tube of the type provided with contacts 26 at one end only and is diagonally positioned inside the envelope 19 so as to reduce the cartridge size. The contacts 26 are connected to a socket or corresponding contacts 25 formed on the inside of the housing 1, and the arrangement is such that the power supply to the lamp is removed unless the cartridge is properly positioned in the aperture 10.

In accordance with another embodiment of the invention, one or both UV stopping devices 15, 16 can be realised as a durable UV stopping device, such as a so called optic labyrinth, i.e. a meander path adapted to cancel the UV radiation through multiple reflections onto an adsorbing inner surface, or through suitable means, and located within the housing, e.g. on the inside of the grid 5 or 8.

The disposable cartridge 2 further comprises a system (of known type comprising for example a microchip and not shown in the Figures) capable of recording and counting the time of use of the unity, as well to supply data for identifying the cartridge and check its compatibility with the device. Advantageously a magnetised strip 21 is secured to the inner bottom surface of the housing and co-operates with a metal strip 22 on the cartridge surface to secure the coupling between the housing and the cartridge.

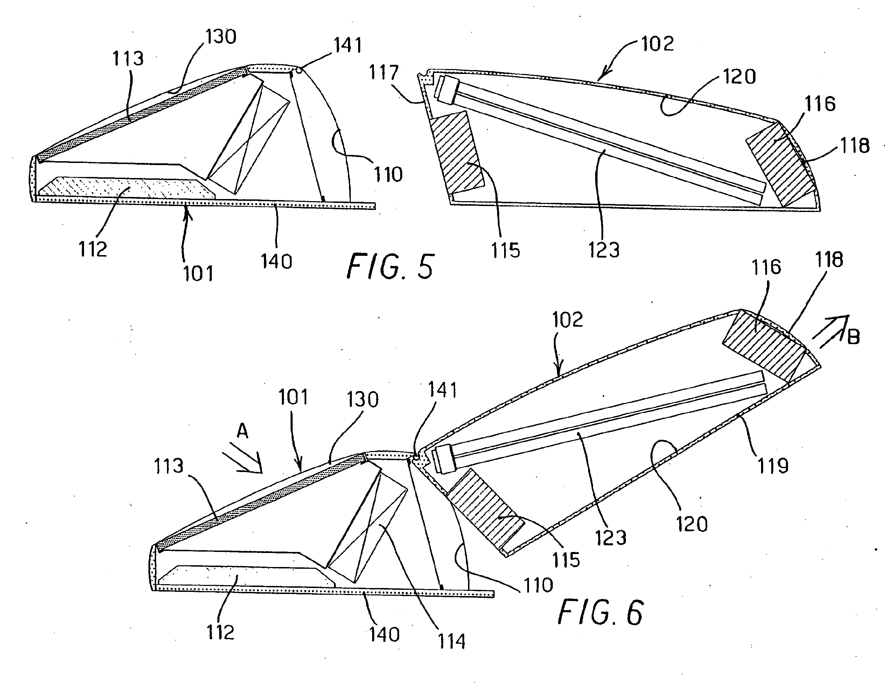

According to the embodiment illustrated in Figs. 5 and 6, the support member 101 is formed with an opening 110, onto which a cantilevered disposable cartridge 102 is applied, said cartridge containing germicidal lamps 123 and being adapted to be secured to one end of the support frame 101.

The frame 101 is formed as a supporting structure provided with a base 140 that incorporates means (shown not) to secure the apparatus to a wall, and provides for an articulated joint 141 into which the cartridge is fitted, such joint allowing a partial rotation of the cartridge. The support member 101 includes a assembly 112 incorporating a power supply to be connected to the electric mains or other source of electric power, and an electronic control circuitry, a first coarse filter or pre-filter 113 that covers the opening 130 of the support member and two helical fans 114 disposed between the filter 113 and the other opening 110 in the support member.

The cartridge 102 is made of a relatively rigid material, such as plastics, cardboard, wood or the like, in case with a reflecting inner surface 120, for instance thanks to a reflecting coating or liner, such as an aluminium film. The cartridge 102 has an elongated shape and comprises a cartridge housing 119 provided with two openings at the ends thereof, respectively an opening 117 for the inlet and an opening 118 for the outlet of a flow of air schematically indicated by the arrows A and B. At least one of the openings 117, 118 is equipped with means 115, 116 allowing the passage of the air flow but stopping the germicidal radiation, such as for example a dark filter or an activated charcoal filter and similar apparatuses. These filters are disposed together with the cartridge when this latter is replaced.

## Claims

1. An ultraviolet sterilisation apparatus comprising a housing (1; 101) and a replaceable lamp cartridge (2; 102) containing at least one ultraviolet lamp (23; 123), said housing including an opening (30) for the inlet of an air flow to be sterilised and an opening (31) for the outlet of the sterilised air flow, means (14) being provided for generating said air flow, said means (14) for generating said air flow being located inside said housing (1; 101)
**characterised in that**
said cartridge (2; 102) is a disposable component comprising an envelope (19; 119) formed with a reflecting inner surface (20) and two openings (17, 18; 117, 118) for the entry and exit of said air flow, at least one of said openings (17, 18, 117, 118) being provided with means (15, 16; 115, 116) that allow the passage of the air but stops the UV germicidal radiation emitted by said lamp(s) and **in that** said cartridge (2; 102) is a pre-assembled package removably fixable to said housing (1; 101) in order to allow the replacement of the whole cartridge comprising all the components thereof (23, 16, 16; 123, 115, 116).

2. A sterilization apparatus according to claim 1, **characterized in that** said housing (1; 101) provides for an aperture (10) into which at least a portion of said lamp cartridge (2, 102) is inserted.

3. A sterilization apparatus according to claim 1, **characterized in that** both of the two openings (17, 18; 117, 118) of said cartridge (2; 102) are provided with means (15, 16; 115, 116) that allow the passage of the air but stop the germicidal radiation, each one of said means (15, 16; 115, 116) being selected from the group consisting of a dark filter an activated charcoal filter, an optic labyrinth.

4. A sterilization apparatus according to claim 1, **characterized in that** said housing (1; 101) further contains a coarse dust filter forming (13; 113) a wall of said housing, and **in that** said means for generating an air now comprises at least one fan (14; 114) interposed between said dust filter (13; 113) and said cartridge (2; 102).

5. A sterilization apparatus according to claim 1, **characterized in that** said housing (1; 101) is a supporting frame incorporating means for fixing the whole sterilization apparatus to a wall (6).

6. A sterilization apparatus according to claim 1, **characterized in that** said cartridge envelope (19; 119) is of a material selected from the group consisting of plastics, cardboard, wood, the inner surface of which is covered or lined by a reflecting film of aluminium.

7. A sterilization apparatus according to claim 1, **characterized in that** said cartridge envelope (19; 119) comprises a plurality of electric contacts (26) for the UV lamp (23; 123) disposed inside said envelope (19; 119), said contacts (26) being connected to a socket or corresponding contacts (25) formed oh said housing or supporting frame (1; 101).

8. A sterilization apparatus according to claim 7, **characterized in that** said housing or supporting frame (1; 101) incorporates a power supply (11) for feeding the apparatus, and a control circuitry (12) realized with one or more electronic cards, said housing incorporating control and adjustment buttons (3), and a display (4) showing the operating hours of said cartridge (2; 102).

9. A sterilization apparatus according to claim 1, **characterized in that** it further comprises a magnetized strip (21) secured to the inner bottom surface of the housing (1) and cooperating with a metal strip (22) on said cartridge (2) surface to secure the coupling between the housing and the cartridge, and a flat panel (7) adapted to carry an advertising message which is visible when the cartridge (2) is fitted within said housing (1).

10. A sterilization apparatus according to claim 1, **characterized in that** the lamp is a tube (23; 123) and it is diagonally positioned inside the envelope (19; 119) so as to reduce the cartridge (2; 102) size.

## Patentansprüche

1. Vorrichtung zur Sterilisierung durch ultraviolette Strahlung, beinhaltet ein Gehäuse (1, 101) und einen Einsatz für austauschbare Lampen (2, 102) mit mindestens einer ultraviolett ausströmenden Lampe (23, 123), besagte Hülle beinhaltet eine Eingangsöffnung (30) für den zu sterilisierenden Luftfluss und eine Ausgangsöffnung (31) für die sterilisierte Luft, denn sie verfügt über die Mittel (14) zur Erzeugung der besagten Luftströmung, die besagten Mittel (14) zur Erzeugung der besagten Luftströmung befinden sich im besagten Gehäuse (1; 101), Vorrichtung **dadurch gekennzeichnet, dass** es sich bei dem besagten Einsatz (2, 102) um ein Einweg-Bestandteil handelt, das ein Gehäuse (19; 119) mit innerer reflektierender Oberfläche (20; 120) und zwei Öffnungen (17, 18; 117, 118) für den Eintritt und den Austritt der Luftströmung besitzt; mindestens eine der besagten Öffnungen (17, 18; 117, 118) verfügt über die Mittel (15, 16; 115, 116), die den Durchgang von Luft ermöglichen, aber die keimtötende UV-Strahlung, welche durch die besagte Lampe erfolgt, verhindern, und durch die Tatsache, dass der besagte Einsatz (2; 102) ein am Gehäuse (1; 101) vor-montiertes, herausnehmbar angebrachtes Ganzes ist, das den Ersatz des gesamten Einsatzes, der alle Bauteile des Einsatzes (23, 15, 16; 123, 115, 116) beinhaltet, ermöglicht.

2. Sterilisierungsvorrichtung, gemäß den Ansprüchen 1, **dadurch gekennzeichnet, dass** das besagte Gehäuse (1) eine weitere Öffnung (10) vorsieht, in der mindestens ein Teil des besagten Einsatzes für Lampen (2; 102) eingefügt ist.

3. Vorrichtung zum Sterilisieren, gemäß den Ansprüchen 1, **dadurch gekennzeichnet, dass** beide Öffnungen (17, 18; 117, 118) des besagten Einsatzes für Lampen (2; 102) über Mittel (15, 16; 115, 116) verfügen, die den Durchlass der Luft ermöglichen, aber die keimtötende Strahlung blockieren. Jedes Einzelne der besagten Mittel (15, 16; 115, 116), wird aus der Gruppe, die einen dunklen Filter, einen Aktivkohlefilter und ein optisches Labyrinth beinhaltet, gewählt.

4. Vorrichtung zum Sterilisieren, gemäß den Ansprüchen 1, **dadurch gekennzeichnet, dass** das besagte Gehäuse (1; 101) über einen Filter für grobe Staubpartikel (13; 113) verfügt, der eine Wand des besagten Gehäuses bildet, und dass die besagten Mittel zur Erzeugung einer Luftströmung über mindestens einen Ventilator (14; 114) verfügen, der zwischen dem besagten Staubfilter (13; 113) und dem besagten Einsatz (2; 102) liegt.

5. Vorrichtung zum Sterilisieren, gemäß den Ansprüchen 1, **dadurch gekennzeichnet, dass** es sich bei dem besagten Gehäuse (1; 101) um eine Halterungsstruktur handelt, welche über die Mittel zur Anbringung der genannten Vorrichtung an eine Wand (6) verfügt.

6. Vorrichtung zum Sterilisieren, gemäß den Ansprüchen 1, **dadurch gekennzeichnet, dass** das besagte Gehäuse (19; 119) des Einsatzes aus Material aus der Gruppe, welche Plastik, Karton und Holz beinhaltet, besteht, dessen innere Oberfläche auf der inneren Seite vollkommen überzogen oder mit einem reflektierenden Aluminiumfilm ausgekleidet ist.

7. Vorrichtung zum Sterilisieren, gemäß den Ansprüchen 1, **dadurch gekennzeichnet, dass** die besagte Hülle des Einsatzes (19; 119) über eine Vielzahl elektrischer Kontakte (26) für die UV-Lampe (23; 123) verfügt, die im besagten Gehäuse (19) angeordnet ist; die besagten Kontakte sind an eine Steckdose oder an entsprechende Kontakte (25) angeschlossen, die sich im genannten Gehäuse oder einer Halterungsstruktur befinden (1; 101).

8. Vorrichtung zum Sterilisieren, gemäß den Ansprüchen 7, **dadurch gekennzeichnet, dass** das besagte Gehäuse oder die Halterungsstruktur (1; 101) über ein Netzgerät (11) zur Speisung der Vorrichtung verfügt, und eine Kontroll-Schalttechnik (12), mit einem oder mehreren Schaltkreisen, das besagte Gehäuse (1), das über Kontroll- und Regelschalter (3) und ein Sichtgerät (4), das die Dauer der Inbetriebnahme des Einsatzes ((2; 102) anzeigt, verfügt.

9. Vorrichtung zum Sterilisieren, gemäß den Ansprüchen 1, **dadurch gekennzeichnet, dass** sie über einen magnetisierten Streifen (21) verfügt, der auf der unteren Innenoberfläche des Gehäuses (1) befestigt ist und mit einem Metallstreifen (22), der sich auf der Oberfläche des besagten Einsatzes befindet, zusammen arbeitet, um eine Verbindung zwischen dem Gehäuse und dem Einsatz zu garantieren, und **dadurch**, dass ein flaches Paneel (7), das geeignet ist, einen Werbetext zu tragen, sichtbar wird, wenn der Einsatz (2) an das besagte Gehäuse (1) angebracht wird.

10. Vorrichtung zum Sterilisieren, gemäß den Ansprüchen 1, **dadurch gekennzeichnet, dass** die Lampe ein Rohr (23; 123) ist und diagonal im Inneren des Gehäuses (19; 119) so angebracht wird, dass die Größe des Einsatzes (2; 102) reduziert wird.

## Revendications

1. Dispositif de stérilisation au moyen d'un rayonnement ultraviolet comprenant une enveloppe (1, 101) et une cartouche pour lampes remplaçable (2, 102), qui contiens au moins une lampe émettrice d'ultraviolets (23, 123), ladite enveloppe comprenant une ouverture (30) d'entré d'un flux d'air à stériliser et un'ouverture (31) de sortie de l'aire stérilisée, étant prévus des moyens (14) pour générer ledit flux d'air, lesdits moyens (14) pour générer ledit flux d'air étant disposés dans ladite enveloppe (1, 101), dispositif **caractérisée en ce que** ladite cartouche (2, 102) est un composant jetable qui comprend une enveloppe (19,119) formée avec une surface interne réfléchissante (20,120) et deux ouvertures (17, 18; 117, 118) pour l'entré e la sortie dudit flux d'air, au moins une desdites ouvertures (17, 18; 117, 118) étant douée de moyens (15, 16; 115, 116) qui permettent le passage de l'air mais ils bloquent la radiation germicide UV émise par ladite lampe et que ladite cartouche (2 ; 102) est un ensemble pré-assemblé réversiblement fixable à ladite enveloppe (1, 101) de manière à permettre la substitution de ladite cartouche comprenant tous les composants de la même (23, 15, 16; 123, 115, 116).

2. Dispositif de stérilisation selon la revendication 1 **caractérisé en ce que** ladite enveloppe (1) prévois une autre ouverture (10) dans laquelle est insérée au moins une portion de ladite cartouche pour lampes (2; 102).

3. Dispositif de stérilisation selon la revendication 1 **caractérisé en ce que** les deux ouvertures (17, 18; 117, 118) de ladite cartouche pour lampes (2; 102) sont douées de moyens (15, 16; 115, 116) qui permettent le passage de l'air mais ils bloquent la radiation germicide, chacun desdits moyens (15, 16; 115, 116) étant choisi par le groupe comprenant un filtre obscur, un filtre de charbon actif, un labyrinthe optique.

4. Dispositif de stérilisation selon la revendication 1 **caractérisé en ce que** ladite enveloppe (1; 101) contiens en outre un filtre pour poudres grossières (13; 113) qui forme une paroi de ladite enveloppe, et que lesdits moyens pour générer un flux d'air comprennent au moins un ventilateur (14; 114) interposé entre ledit filtre pour poudres (13, 113) et ladite cartouche (2; 102).

5. Dispositif de stérilisation selon la revendication 1 **caractérisé en ce que** ladite enveloppe (1 ;101) est une structure de support qui englobe des moyens pour fixer ledit appareillage à une paroi (6).

6. Dispositif de stérilisation selon la revendication 1 **caractérisé en ce que** ladite enveloppe (19;119) de la cartouche est d'un matériel sélectionné par le groupe comprenant plastique, carton, bois, la surface interne du quel est entièrement recouverte ou revêtue par un film réfléchissant en aluminium.

7. Dispositif de stérilisation selon la revendication 1 **caractérisé en ce que** dite enveloppe de la cartouche (19; 119) comprend une pluralité de contacts électriques (26) pour la lampe UV (23; 123) disposée dans ladite enveloppe (19); lesdits contacts étant reliés à une prise ou à contacts correspondants (25) formés dans ladite enveloppe ou structure de support (1; 101).

8. Dispositif de stérilisation selon la revendication 7 **caractérisé en ce que** dite enveloppe de la cartouche ou structure de support (1; 101) englobe un alimentateur (11) pour alimenter le dispositif, et un circuit de contrôle (12) comprenant une ou plusieurs fiches électroniques, dite enveloppe (1) comprenant boutons-poussoirs de contrôle et régulation (3), et un visualiseur (4) qui montre les heures d'emploie de la cartouche (2, 102).

9. Dispositif de stérilisation selon la revendication 1 **caractérisé en ce que** comprend une bande magnétisée (21) fixée sur la surface inferieure interne de l'enveloppe (1) et laquelle coopère avec une bande de métal (22) placée sur la surface de ladite cartouche pour assurer le couplage entre l'enveloppe et la cartouche, et que un panneau plat (7), apte a porter un message publicitaire, est visible lorsque la cartouche (2) est appliquée à ladite enveloppe (1).

10. Dispositif de stérilisation selon la revendication 1 **caractérisé en ce que** la lampe est un tube (23, 123) et elle est positionnée diagonalement à l'intérieur de l'enveloppe (19; 119) de façon à réduire les dimensions de la cartouche (2; 102).
